# EUROPEAN PATENT SPECIFICATION

(11) **EP 2 530 501 B1**
(45) Date of publication and mention of the grant of the patent: **24.08.2016**
(21) Application number: 10844806.9
(22) Date of filing: 22.11.2010
(51) Int. Cl.: G02B 6/00

(54) **PLASTIC SCINTILLATOR, AND SCINTILLATION DETECTOR AND MEDICAL DIAGNOSTIC IMAGING EQUIPMENT USING SAME**
KUNSTSTOFFSZINTILLATOR UND SZINTILLATIONSDETEKTOR SOWIE MEDIZINISCH-DIAGNOSTISCHES BILDGEBUNGSGERÄT DAMIT
SCINTILLATEUR PLASTIQUE, DÉTECTEUR À SCINTILLATION ET ÉQUIPEMENT D'IMAGERIE DIAGNOSTIQUE MÉDICALE UTILISANT UN TEL DÉTECTEUR

(30) Priority: 28.01.2010 KR 20100008138
(43) Date of publication of application: 05.12.2012
(73) Proprietor: Suh, Jun-Suhk, Donggu Daegu 41061 (KR); Park, Hyeun-Suk, Donggu Daegu 41061 (KR)
(72) Inventor: Suh, Jun-Suhk, Donggu Daegu 41061 (KR); Park, Hyeun-Suk, Donggu Daegu 41061 (KR)
(74) Representative: Tomlinson, Kerry John
(86) International application number: PCT/KR2010/008218
(87) International publication number: WO 2011/093582

(56) References cited:
- EP-A2- 1 921 465
- EP-A2- 1 921 465
- WO-A2-2008/107808
- WO-A2-2009/024895
- WO-A2-2009/083852
- JP-A- 9 236 669
- JP-A- 9 236 669
- BEDDAR ET AL: "Plastic scintillation dosimetry and its application to radiotherapy", RADIATION MEASUREMENTS, ELSEVIER, AMSTERDAM, NL, vol. 41, 1 December 2006 (2006-12-01), pages S124-S133, XP028073735, ISSN: 1350-4487, DOI: 10.1016/J.RADMEAS.2007.01.002 [retrieved on 2006-12-01]
- IKHLEF A ET AL: "X-ray imaging and detection using plastic scintillating fibers", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 442, no. 1-3, 1 March 2000 (2000-03-01), pages 428-432, XP004193531, ISSN: 0168-9002, DOI: 10.1016/S0168-9002(99)01268-1
- BEDDAR ET AL.: 'Plastic scintillation dosimetry and its application to radiotherapy' RADIATION MEASUREMENTS vol. 41, 01 December 2006, pages S124 - S133, XP028073735

## Description

### BACKGROUND

### 1. Technical Field

The present invention relates to a scintillator of a scintillation detector that detects high energy particles and a method of incorporating a scintillator in medical diagnostic imaging equipment, more specifically to a scintillator that emits light by detecting high energy particles generated by an object examined by a common medical diagnostic imaging equipment and a scintillation detector and a positron emission tomography device, which is such a piece of medical diagnostic imaging equipment, using such a scintillator and a photomultiplier.

### 2. Background Art

Medical diagnostic imaging equipment commonly includes computed tomography (CT), magnetic resonance imaging (MRI) and the like. Such imaging technologies are increasingly used for a more accurate examination by spotting a region having growing tissues to identify a thrombus, a scar, dead cancer tissue and the like from living tissues. In the medical device industry, which has recently attracted more attention, the market size of the medical diagnostic imaging equipment has reached nearly 50% of the entire medical device markets.

As every kind of diagnostic imaging equipment has been accomplishing faster diagnosing time, real-time diagnosis and multi-dimensional imaging (e.g., 3D imaging and 4D imaging), various diagnostic methods have been developed for application in the medical diagnostic imaging equipment. However, not only does it take a great length of time to perform diagnosis using the medical diagnostic imaging equipment (e.g., 12 hours for full-body CT, 24 hours for full-body MRI, and 1 hour for full-body PET), but the examination cost is too high for the general public to afford, restraining a wide use of the medical diagnostic imaging equipment.

A cause of the above problems is the scintillator, which is an essential element that emits light by being in contact with high energy particles during an examination, and of which a crystal scintillator is commonly used. The crystal scintillator, which is expensive and processing of which is difficult and costly, is a main cause of raising the price of medical diagnostic imaging equipment and increasing the examination time due to its difficulty of constituting in a wide area.

Bedder et al., "Plastic scintillation dosimetry and its application to radiotherapy", Radiation Measurements, Elsevier, Amsterdam, Netherlands, vol. 41, 1 December 2006, pages S124-S133 discloses a scintillation detector system comprising a plastic scintillator, fiber light guides and photomultiplier tubes.

WO 2009/083852 discloses a radiation-sensitive detector including a photosensor and a scintillator.

WO 2008/107808 discloses a positron emission tomography device comprising an array of scintillator material coupled to an array of photodetectors.

### SUMMARY

The invention provides a positron emission tomography device, which is medical diagnostic imaging equipment, using a scintillation detector, the positron emission tomography device configured to image a detection signal detected using the scintillation detector and comprising: a scintillator formed in a prismatic structure having a polygonal cross-section; a reflecting film formed on an external surface of the scintillator; and a silicon photomultiplier coupled to the scintillator, and characterised in that: the scintillator is a plastic scintillator made of a plastic material; the plastic scintillator comprises a hollow section formed in a central part of the plastic scintillator, and the positron emission tomography device further comprises an optical fiber inserted into the hollow section; and one side of the optical fiber is directly connected to the silicon photomultiplier.

To overcome the limitations of structural improvement for enhancement of detection efficiency due to processing difficulty and high material costs caused by using the conventional scintillator, the present invention provides a plastic scintillator and a scintillation detector and medical diagnostic equipment using the plastic scintillator that can shorten the examination time and lower the manufacturing cost dramatically by utilizing a scintillator having a same effect and using a more economical material.

To achieve the above object, the present invention can use a plastic scintillator to maximally reduce a gap between scintillators by allowing the scintillators to have various cross-sectional shapes, constitute the scintillator by including optical fiber, which is an effective detecting material, to enhance detectability, and dramatically increase an area where the scintillator is constituted when utilized in a medical diagnostic imaging equipment.

With the present invention, the cost of raw material becomes remarkably lower than the conventional scintillator, and it becomes much easier for processing, thereby allowing for more efficient configuration and processing for detection of a high energy particle. Ultimately, the detection area of the medical diagnostic imaging equipment can be dramatically larger to reduce the detection time, allowing for increased convenience for users and supply at lower costs.

### BRIEF DESCRIPTION OF THE DRAWINGS

FIG. 1 shows conventional medical diagnostic imaging equipment.
FIG. 2 is a perspective view illustrating an embodiment of the present invention.

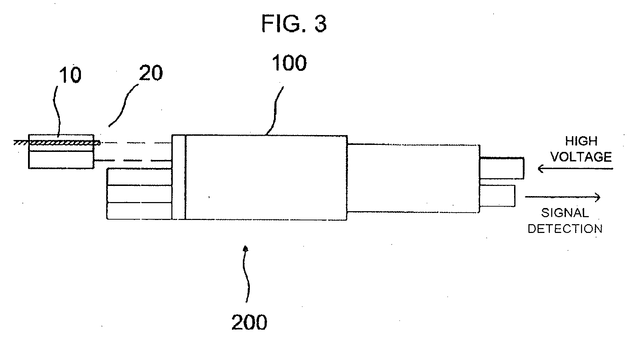

FIG. 3 is a perspective view illustrating some embodiments of the present invention.
FIG. 4 is a cross-sectional view illustrating an embodiment of the present invention.
FIG. 5 is an exploded view illustrating an embodiment of the present invention.
FIG. 6 is a perspective view illustrating some embodiments of the present invention.
FIG. 7 shows a configuration of an embodiment of the present invention.
FIG. 8 is a cross-sectional view illustrating an embodiment of the present invention.

### DETAILED DESCRIPTION

Hereinafter, some embodiments of the present invention will be described in detail with reference to the accompanying drawings.

Several medical diagnostic imaging equipment have been developed, using positron emission tomography (PET). These kinds of medical diagnostic imaging equipment detects a high energy particle generated at a particular region with a scintillator (a crystal exhibiting scintillation when struck by a particle), amplifies the high energy particle through a photomultiplier, converts the high energy particle into, and images a photocurrent detection signal to display the particular region where a problem occurs. As described above in the background art, most of these kinds of medical diagnostic imaging equipment are very expensive and thus are hardly utilized in a popular fashion. One of the causes of this shortcoming is the costly scintillator.

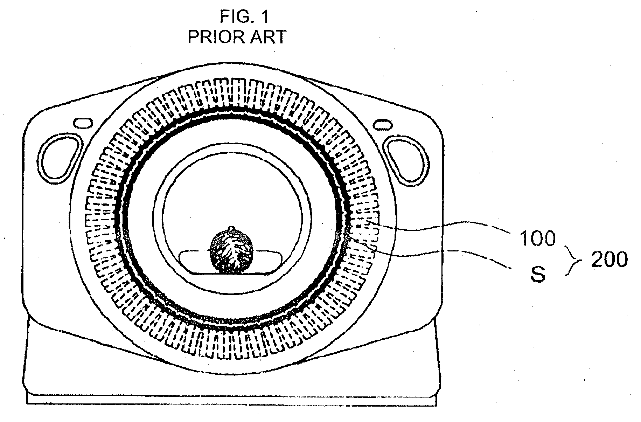

In the conventional medical diagnostic imaging equipment, a photomultiplier 100 has a scintillator (S) inserted therein. As illustrated in FIG. 1, a plurality of the scintillators (S) are inserted into the photomultiplier 100 to form a scintillation detector 200, which is arranged on a main body of the medical diagnostic imaging equipment to surround a cross-section of an examined object.

Used for the scintillator arranged in the scintillation detector is a crystal, but a highly pure crystal (BGO and various kinds of crystal) requires a long time of growth and is difficult to manufacture, making it costly to process and utilize the crystal for the scintillator of a medical diagnostic imaging equipment.

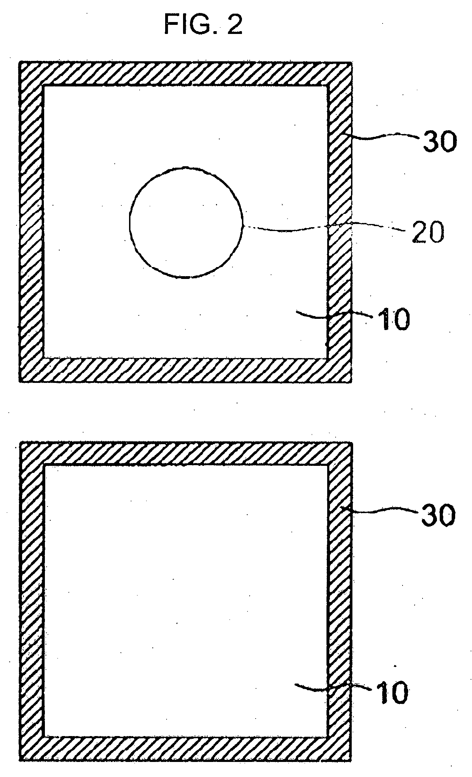

In the present invention, the conventional crystal (such as BGO) is not utilized as the scintillator of the medical diagnostic imaging equipment, but as illustrated in FIG. 2, a plastic scintillator 10 and optical fiber 20 constituted therein are provided to have the same effectiveness as the conventional crystal but with a significant economical effect.

By using the plastic scintillator 10 for the scintillator constituted in the scintillation detector, the performance and detecting effect of the scintillator in accordance with the present invention is unchanged from the conventional crystal scintillator, but is so easy to process that it can be fabricated in various shapes at incomparably low costs.

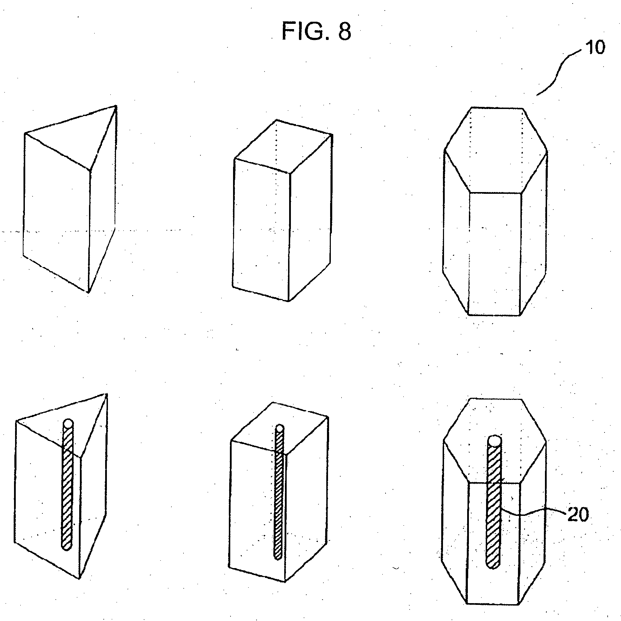

It is so difficult and costly to process the conventional crystal scintillator that the scintillator is formed in the shape of a hexahedron, in which a cross-section on a side of detecting the high energy particle is close to a square, and bound with the photomultiplier. However, as illustrated in FIG. 8, with the plastic scintillator in accordance with an embodiment of the present invention, it is possible to form a cross-section on a side of detecting the high energy particle in the shapes of various polygons, such as a triangle, a rectangle, a pentagon, a hexagon, a heptagon, an octagon, etc.

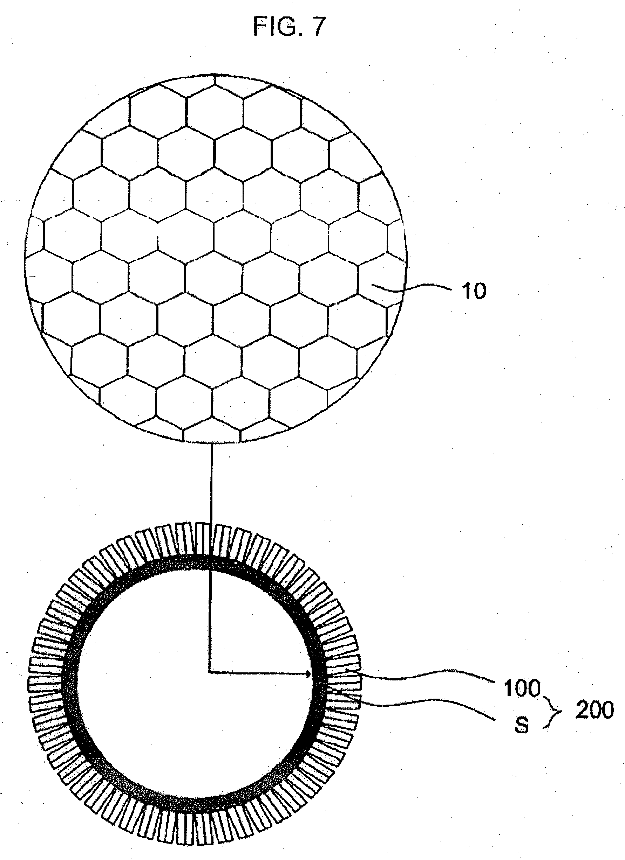

In the present invention, the effectiveness of detection can be enhanced by minimizing a gap among the scintillators, as the scintillation detector 200 is constituted with the photomultiplier 100 in which the plastic scintillator 10 in the shape of a hexagon is used. That is, as illustrated in FIG. 7, in the case that the scintillation detector 200 of medical diagnostic imaging equipment is constituted with the plastic scintillator 10 having a hexagonal cross-section, which is commonly referred to as a honeycomb structure, gaps that can occur among the scintillators 10 are relatively smaller than those of other cross-sectional shapes, making it possible to detect the high energy particles more efficiently.

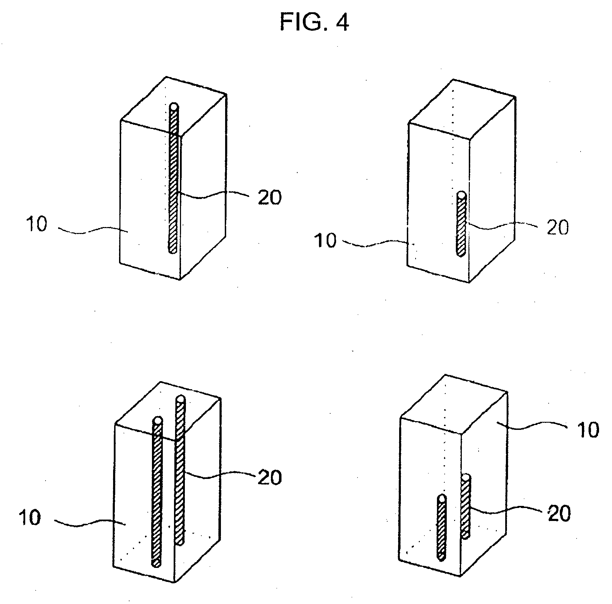

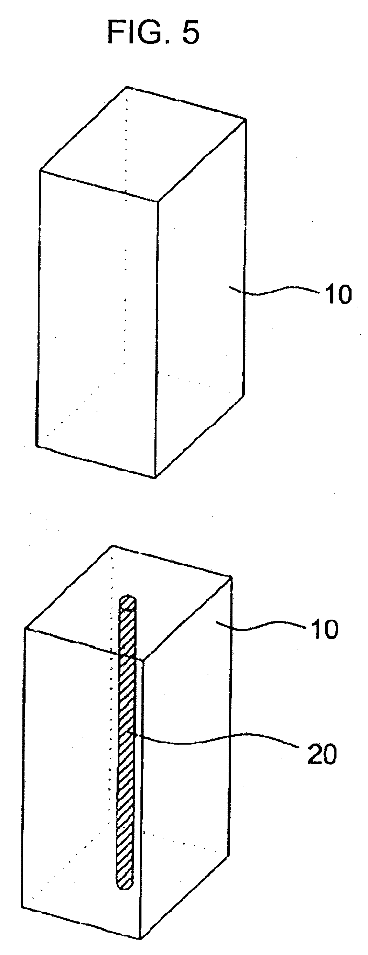

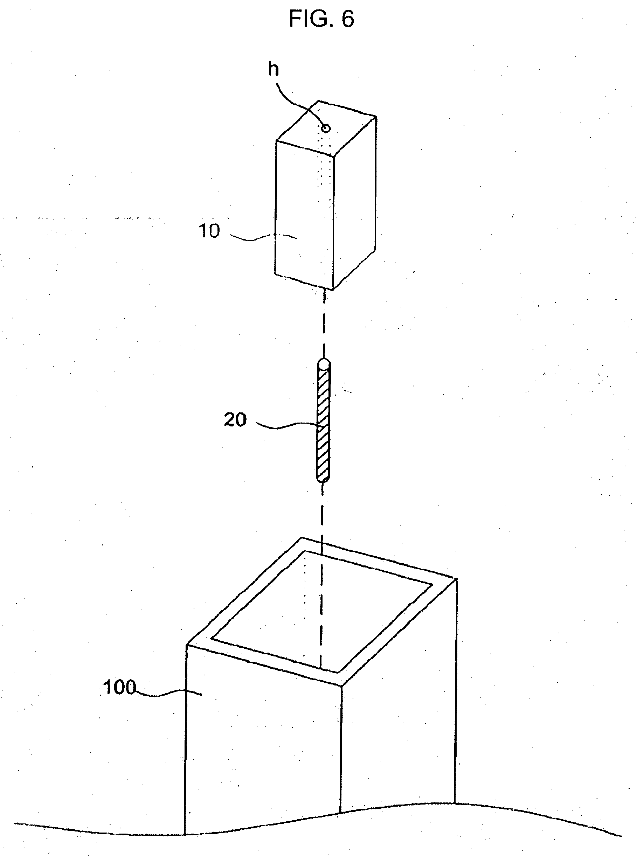

As illustrated in FIGS. 2, 4, 5, 6 and 8, it is possible to process and constitute this kind of plastic scintillator 10 in a highly efficient form and thus to form a hollow section h. Although it is possible to bind the plastic scintillator 10 as is with the photomultiplier 100, an integrated plastic scintillator 10' can be constituted by forming a hollow section h inside a central part thereof and inserting the optical fiber 20 into the hollow section h in order to collect the light emitted from the plastic scintillator and transfer the light to the photomultiplier 100. In such a configuration, it is possible to allow the optical fiber 20 to penetrate through the plastic scintillator 10 or allow the optical fiber 20 to penetrate the scintillator 10 where the scintillator 10 makes contact with the photomultiplier 100 and penetrate the scintillator 10 or be formed not to be exposed to an outside on the side of detecting the high energy particle. Moreover, it is possible to form a plurality of hollow sections h and insert a plurality of optical fiber 20 accordingly, or use a fiber optic core selectively or clad an external part of the core (not shown).

In constituting the scintillation detector 200 applied with the plastic scintillator 10 in which the optical fiber 20 is formed, one side of the optical fiber 20 can be directly connected with the photomultiplier 100 in order to enhance the effect of detection. It shall be appreciated that, in the case of a plastic scintillator 10 that does not include optical fiber 20, its cross-section can be bound to the photomultiplier 100 in a conventional way.

As illustrated in FIG. 2, the light can be better collected by forming a reflecting film on an external surface of the plastic scintillator 10, in which case the reflecting film 30 can have a lower refractive index than a conventional plastic scintillator 10.

Although there can be various methods of forming a plastic scintillator, in an embodiment of the present invention, fluorescent additives, which can be classified into a primary fluorescent additive and a secondary fluorescent additive, can be used for the plastic scintillator 10. Used as the primary fluorescent additive can be p-terphenyl (PT) or 2,5-dephenyloxazole (PPO). Used as the secondary fluorescent additive, i.e., a wavelength transfer agent, can be POPOP or 4-bis(2-Methylstyryl) benzene (bis-MSB).

Used as the fluorescent additive for the optical fiber can be K27, BBQ(7H-benzimidazo[2,1 -a]benz[de]isoquinoline-7-one) of National Diagnostics, or Lumogen of BASF. Accordingly, the fluorescent additive in the color of red, orange, yellow, green, blue, purple or pink can be added according to the usage of detection to use an entire wavelength between 200 nm and 900 nm, thereby allowing for use in the conventional photomultiplier tube (PMT), silicon photomultiplier (SIPM) or multi pixel photon counter. (MPPC).

Used for a material to clad the optical fiber can be poly methyl metha acrylate (PMMA), of which the refractive index is 1.59 and the density is 1.19, in the case that PS is used as a core of the optical fiber for primary cladding of the optical fiber. In addition, any material (e.g., PTFE or PEFE) having the refractive index that is smaller than that of PMMA can be used for secondary cladding over the primary cladding, or the secondary cladding can be optionally omitted.

In the case that PMMA is used for the core of the optical fiber, it is preferable that PTFE or PEFE, of which the refractive index is smaller than that of PMMA, is used for the cladding. That is, it is preferable that aluminum or titanium dioxide (TiO₂) is used for the reflecting film located outside a scintillating cell.

## Claims

1. A positron emission tomography device, which is medical diagnostic imaging equipment, using a scintillation detector (200), the positron emission tomography device configured to image a detection signal detected using the scintillation detector and comprising:
a scintillator (10) formed in a prismatic structure having a polygonal cross-section;
a reflecting film formed on an external surface of the scintillator; and
a silicon photomultiplier (100) coupled to the scintillator,
and **characterised in that**:
the scintillator is a plastic scintillator made of a plastic material;
the plastic scintillator comprises a hollow section formed in a central part of the plastic scintillator, and the positron emission tomography device further comprises an optical fiber (20) inserted into the hollow section; and
one side of the optical fiber (20) is directly connected to the silicon photomultiplier (100).

2. The positron emission tomography device of claim 1, wherein the reflecting film comprises aluminum or titanium dioxide (TiO₂).

3. The positron emission tomography device of claim 1 or 2, wherein the plastic scintillator comprises a primary fluorescent additive made of p-terphenyl (PT) or 2,5-dephenyloxazole (PPO) and a wavelength transfer agent.

4. The positron emission tomography device of claim 3, wherein the wavelength transfer agent is made of POPOP or 4-bis(2-Methylstyryl) benzene (bis-MSB).

## Patentansprüche

1. Eine Vorrichtung für Positronen-Emissions-Tomographie, welche ein medizinisch-diagnostisches Bildgebungsgerät ist, unter Verwendung eines Szintillationsdetektors (200), wobei die Vorrichtung für Positronen-Emissions-Tomographie konfiguriert ist, um ein unter Verwendung des Szintillationsdetektors detektiertes Detektionssignal abzubilden und aufweisend:
einen in einer prismatischen Struktur ausgebildeten Szintillator (10) mit einem polygonalen Querschnitt;
einen auf einer Außenoberfläche von dem Szintillator ausgebildeten reflektierenden Film; und
einen mit dem Szintillator verbundenen Silizium-Photovervielfacher (100),
und **dadurch gekennzeichnet, dass**:
der Szintillator ein aus einem Kunststoffmaterial hergestellter Kunststoffszintillator ist;
wobei der Kunststoffszintillator ein in einem zentralen Teil von dem Kunststoffszintillator ausgebildetes Hohlprofil aufweist und die Vorrichtung für Positronen-Emissions-Tomographie weiter eine in das Hohlprofil eingefügte Lichtleitfaser (20) aufweist; und
eine Seite von der Lichtleitfaser (20) mit dem Silizium-Photovervielfacher (100) direkt verbunden ist.

2. Die Vorrichtung für Positronen-Emissions-Tomographie nach Anspruch 1, wobei der reflektierende Film Aluminium oder Titandioxid (TiO₂) aufweist.

3. Die Vorrichtung für Positronen-Emissions-Tomographie nach Anspruch 1 oder 2, wobei der Kunststoffszintillator ein primäres fluoreszierendes Additiv, das aus P-Terphenyl (PT) oder 2,5-Dephenyloxazol (PPO) hergestellt ist, und ein Wellenlängenübertragungsmittel aufweist.

4. Die Vorrichtung für Positronen-Emissions-Tomographie nach Anspruch 3, wobei das Wellenlängenübertragungsmittel aus POPOP oder 4-Bis(2-Methylstyryl) Benzen (Bis-MSB) hergestellt ist.

## Revendications

1. Dispositif de tomographie par émission de positons, qui est un équipement d'imagerie pour diagnostic médical, utilisant un détecteur de scintillation (200), le dispositif de tomographie par émission de positons étant configuré pour imager un signal de détection détecté en utilisant le détecteur de scintillation et comprenant :
un scintillateur (10) formé dans une structure prismatique ayant une section transversale polygonale ;
un film réfléchissant formé sur une surface externe du scintillateur ; et
un photomultiplicateur de silicium (100) couplé au scintillateur,
et **caractérisé en ce que** :
le scintillateur est un scintillateur en plastique fait d'une matière plastique ;
le scintillateur en plastique comprend une section creuse formée dans une partie centrale du scintillateur en plastique, et le dispositif de tomographie par émission de positons comprend en outre une fibre optique (20) insérée dans la section creuse ; et
un côté de la fibre optique (20) est directement relié au photomultiplicateur de silicium (100).

2. Dispositif de tomographie par émission de positons selon la revendication 1, dans lequel le film réfléchissant comprend du dioxyde de titane (TiO₂) ou d'aluminium.

3. Dispositif de tomographie par émission de positons selon la revendication 1 ou 2, dans lequel le scintillateur en plastique comprend un additif fluorescent primaire fait de p-terphényle (PT) ou 2,5-déphényloxazole (PPO) et un agent de transfert de longueur d'onde.

4. Dispositif de tomographie par émission de positons selon la revendication 3, dans lequel l'agent de transfert de longueur d'onde est fait de POPOP ou 4-bis(2-Méthylstyryle) benzène (bis-MSB).
